# EUROPEAN PATENT SPECIFICATION

(11) **EP 1 547 525 B1**
(45) Date of publication and mention of the grant of the patent: **09.11.2011**
(21) Application number: 04030476.8
(22) Date of filing: 22.12.2004
(51) Int. Cl.: A61B 8/12, A61B 1/00

(54) **Ultrasonic endoscope comprising an ultrasonic signal cable connector**
Ultraschallendoskop mit einem Ultraschallsignalkabelverbinder
Endoscope ultrasonique avec connecteur de câble pour les signaux ultrasonore

(30) Priority: 26.12.2003 JP 2003435623
(43) Date of publication of application: 29.06.2005
(73) Proprietor: Olympus Corporation, Tokyo 151-0072 (JP)
(72) Inventor: Saiga, Kazuya, Shibuya-ku, Tokyo (JP)
(74) Representative: von Hellfeld, Axel

(56) References cited:
- EP-A- 0 904 725
- WO-A-99/19213
- GB-A- 2 241 391
- US-A- 5 125 411
- PATENT ABSTRACTS OF JAPAN vol. 2000, no. 08, 6 October 2000 (2000-10-06) & JP 2000 139927 A (OLYMPUS OPTICAL CO LTD), 23 May 2000 (2000-05-23)

## Description

### BACKGROUND OF THE INVENTION

### 1. Field of the Invention

The present invention relates to an ultrasonic endoscope having an ultrasonic connector that connects to ultrasonic measuring equipment and observes the body cavity using ultrasonic wave, and to an ultrasonic signal cable connector device.

### 2. Description of the Related Art

Various types of endoscopes are widely used in the medical field, which are inserted into the body cavity to observe the living-body tissue or perform biopsy or treatment.

One type of such endoscopes is the ultrasonic endoscope. This ultrasonic endoscope has an ultrasonic transducer on the distal end of the insertion portion that is to be inserted in the body cavity. The ultrasonic transducer of this ultrasonic endoscope transmits ultrasonic wave to the living-body tissue, and receives the ultrasonic wave reflected from the living-body tissue. Thus, an ultrasonic tomographic image is generated by the various observation equipment connected to the ultrasonic endoscope, and the living-body tissue can be observed.

This ultrasonic endoscope is provided on the proximal end portion of the ultrasonic signal cable, as described in Japanese Patent Application Publication No. 2000-139927 for example, and has an ultrasonic connector that connects to an ultrasonic measuring equipment. This ultrasonic connector is provided with, for example, four Flexible Printed Circuits (hereafter "FPC"). The four FPCs each are connected to one end of each of multiple groups of signal lines. Further, the other end of each group of the multiple signal lines is connected to piezoelectric elements making up an electronic scanning ultrasonic transducer.

The FPCs and the multiple signal lines divided into groups are connected by the signal lines being soldered to contact pads provided on the FPCs.

Further, an FPC extension connecting pad is made for connecting an extension FPC. One end of this extension FPC can be inserted into the FPC connector of the ultrasonic connector and fixed. This FPC and the extension FPC are connected by the extension FPC connecting pad provided on the FPC being soldered to the pad provided on the extension FPC.

Further, the multiple signal lines extending from the ultrasonic transducer of the ultrasonic endoscope are divided into multiple groups, with an FPC disposed for each group.

A relatively large number, more than several dozen, of the above-described signals lines are built in, although this depends upon the number of piezoelectric elements. Therefore, the ultrasonic signal cable can have a narrower external diameter, the width of the multiple piezoelectric elements can be set narrow, and the electric connecting unit of the signal lines can also be set narrow, resulting in the much narrower diameter of the signal lines, as illustrated in the drawings of Japanese Patent Application Publication No. 2000-139927.

Further, the dimensions of the FPCs connected with the ultrasonic signal cable need to be smaller in diameter than that of the ultrasonic signal cable channel, in order to pass through the ultrasonic signal cable channel which has a small diameter, that is positioned in the insertion unit and so forth.

Therefore, the land width and the land spacing of the contact pad provided on the FPC is made to be very small. Accordingly, the assembly worker needs to have a high degree of skill for soldering the extremely fine signal lines to the contact pad for electric connection.

On the other hand, the FPC connector width and the FPC connector spacing of the multiple FPC connectors provided on the connecting connector of the ultrasonic connector are set relatively wide. Thus, the multiple FPC connectors are arranged so as to avoid contact failure. This ultrasonic connector has an extension FPC for ease of attaching to the connecting connector, and the extension FPC has a contact pad wherein the pad width and pad spacing is set so as to correspond to the FPC connector.

However, in the assembly process, the ultrasonic signal cables with FPCs connected beforehand are inserted through the ultrasonic signal cable channel, and also inserted through a cable insertion hole for the ultrasonic connector, following which extension FPCs are connected by soldering. Accordingly, the assembly worker needs to have a high degree of skill for the complicated soldering of FPCs and extension FPCs within the narrow ultrasonic connector, as well. Also, the soldered portions of the FPC and extension FPC have the problem of readily cracking under external force.

The present invention has been made in light of the above-described situation, and accordingly it is an object thereof to provide an ultrasonic signal cable connector to which are connected flexible wiring boards connected to ultrasonic signal cables which can be easily passed through the narrow ultrasonic signal cable channel, whereby electrical connection work within the narrow ultrasonic connector with little work space is not necessary, and also to provide an ultrasonic endoscope having this ultrasonic signal cable connector.

### SUMMARY OF THE INVENTION

According to the present invention, an ultrasonic signal cable connector device according to claim 1 and an ultrasonic endoscope according to claim 5 are provided.

The above and other objects, features and advantages of the invention will become more clearly understood from the following description referring to the accompanying drawings.

### BRIEF DESCRIPTION OF THE DRAWINGS

Fig. 1 is a description diagram illustrating the configuration of an ultrasonic endoscope according to a first embodiment of the present invention;
Fig. 2 is a cross-sectional diagram illustrating the configuration of an ultrasonic connector according to the first embodiment of the present invention;
Fig. 3 is a plan view illustrating the configuration of an ultrasonic signal cable according to the first embodiment of the present invention;
Fig. 4 is a front view illustrating the configuration of an ultrasonic signal cable connector device according to the first embodiment of the present invention;
Fig. 5 is a back view illustrating the configuration of a connector receptacle provided on the ultrasonic connector that is connected to the ultrasonic signal cable connector device according to the first embodiment of the present invention;
Fig. 6 is a plan view illustrating the attached state of the ultrasonic signal cable connector device and the connector receptacle according to the first embodiment of the present invention;
Fig. 7 is a sectional view illustrating the attached state of the ultrasonic signal cable connector device and the connector receptacle according to the first embodiment of the present invention;
Fig. 8 is a front view illustrating the configuration of the ultrasonic signal cable connector device according to a second embodiment of the present invention;
Fig. 9 is a back view illustrating the configuration of the ultrasonic signal cable connector device according to the second embodiment of the present invention;
Fig. 10 is a cross-sectional view illustrating the configuration of the ultrasonic signal cable connector device according to the second embodiment of the present invention;
Fig. 11 is a plan view illustrating the configuration of a connector receptacle wherein the ultrasonic signal cable connector of the ultrasonic signal cable connector device according to the second embodiment of the present invention is attached;
Fig. 12 is a cross-sectional view illustrating the configuration of the connector receptacle wherein the ultrasonic signal cable connector of the ultrasonic signal cable connector device according to the second embodiment of the present invention is attached;
Fig. 13 is a plan view illustrating the configuration of a connecting board for connecting the ultrasonic signal cable connector of the ultrasonic signal cable connector device and the connector receptacle according to the second embodiment of the present invention;
Fig. 14 is a cross-sectional view illustrating the configuration of a connecting board for connecting the ultrasonic signal cable connector of the ultrasonic signal cable connector device and the connector receptacle according to the second embodiment of the present invention;
Fig. 15 is a plan view illustrating the attached state of the ultrasonic signal cable connector of the ultrasonic signal cable connector device and the connector receptacle according to the second embodiment of the present invention;
Fig. 16 is a cross-sectional view illustrating the attached state of the ultrasonic signal cable connector of the ultrasonic signal cable connector device and the connector receptacle according to the second embodiment of the present invention;
Fig. 17 is a front view illustrating the configuration of the ultrasonic signal cable connector of the ultrasonic signal cable connector device according to a third embodiment of the present invention; and
Fig. 18 is a back view illustrating the configuration of the ultrasonic signal cable connector of the ultrasonic signal cable connector device according to the third embodiment of the present invention.

### DETAILED DESCRIPTION OF THE PREFERRED EMBODIMENTS

### First Embodiment

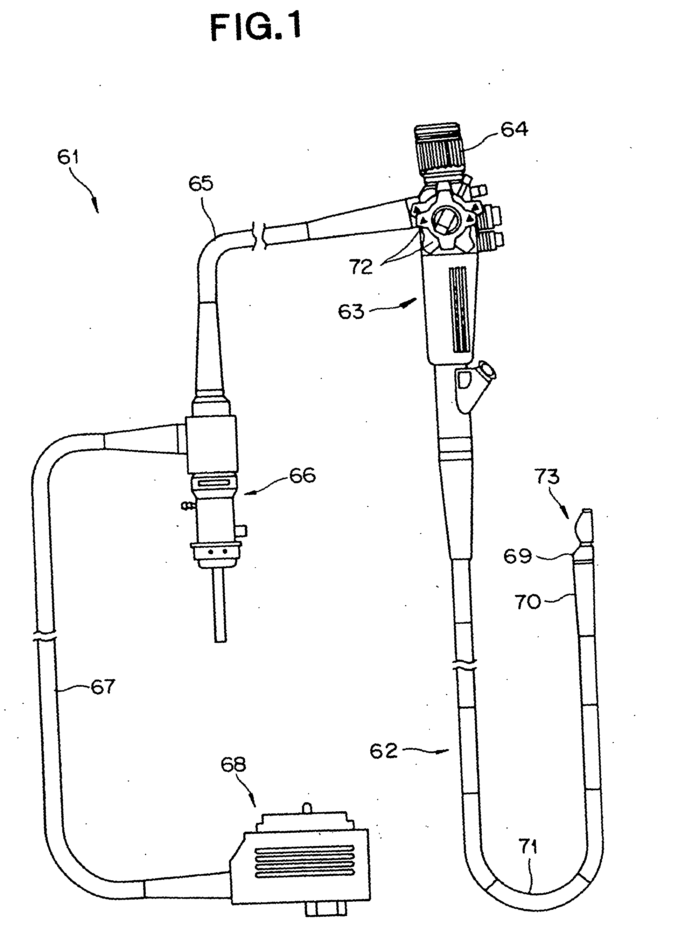

The configuration of the ultrasonic endoscope of the present invention will be described below with reference to the drawings. In Fig. 1, an ultrasonic endoscope 61 primarily comprises an insertion portion 62 that is inserted into the body cavity, an operating unit 63 provided on the rear end of the insertion portion 62, an eyepiece 64 provided on the proximal end of the operating unit 63, a universal cord 65 extending from the operating unit 63, an endoscope connector 66 provided on the proximal end of the universal cord 65 that is connected to a light source device (not shown), and an ultrasonic signal cable 67 that extends from the endoscope connector 66 and has an ultrasonic connector 68 on the distal end thereof. Now, the ultrasonic connector 68 which is this connector unit is connected to an ultrasonic measuring equipment (not shown).

The insertion portion 62 is formed with a small diameter. The insertion portion 62 comprises, in order from the distal end, a hard distal-end portion 69, a bending portion 70, and a long flexible portion 71 that is capable of bending. An ultrasonic transducer unit 73 is located on the distal end side of the hard distal-end portion 69. An electronic scanning ultrasonic transducer, wherein multiple piezoelectric elements that send and receive ultrasonic wave are arrayed, is located on the ultrasonic transducer unit 73.

Disposed on the operating unit 63 are a bending knob 72, an air and water feed button, a suction button, an insertion opening for forceps treatment instruments, and so forth.

The bending knob 72 is an operating knob for bending the bending portion 70 of the insertion portion 62 in the desired direction. The air and water feed button is operated in the moment wherein air or water is fed to the air and water feeding channel provided within the insertion portion 62. The suction button is operated in the moment wherein air or water is suctioned into the suction channel. The insertion opening for forceps treatment instruments is an opening for the forceps channel through which forceps treatment instruments are inserted.

The optical image of the internal body cavity from the object lens provided on the hard distal-end portion 69 of the insertion portion 62 is guided by the image guide, and visually confirmed at the eyepiece 64.

The illumination light from the light source device wherein the endoscope connector 66 is connected is guided through the universal cord 65, and a light guide that is passed through the inside of the operating unit 63 and the insertion portion 62. The illumination light is then emitted from the hard distal-end portion 69 onto the observation portions in the body cavity. The ultrasonic signal cable 67 that extends from the endoscope connector 66 is passed through the universal cord 65, and the ultrasonic signal cable channel (not shown) provided on the insertion portion 62 and the operating unit 63. One end of the ultrasonic signal cable 67 is connected to the electronic scanning ultrasonic transducer of the ultrasonic transducer unit 73, and the other end thereof is connected to the ultrasonic connector 68. The ultrasonic connector 68 is connected to the ultrasonic measuring equipment (not shown) that performs sending/receiving drive control of the ultrasonic wave from the electronic scanning ultrasonic transducer of the ultrasonic transducer unit 73.

Further, the ultrasonic signal cable 67 is connected to the multiple piezoelectric elements of the electronic scanning ultrasonic transducer provided in the ultrasonic transducer unit 73. Multiple signals lines which are signal core lines, to be described below, that send/receive the ultrasonic signal from the piezoelectric elements, pass through the inside of the ultrasonic signal cable 67. These multiple signals lines are grouped in groups with a predetermined number of lines each.

The ends of the multiple signal lines in the ultrasonic signal cable 67 are connected electrically to the multiple piezoelectric elements of the electronic scanning ultrasonic transducer provided on the ultrasonic transducer unit 73. The multiple signal lines are separated into multiple bundles as will be described below, and are inserted in the ultrasonic signal cable channel opening provided on the hard distal-end portion 69 of the insertion portion 62 from the other end side that is not connected with the piezoelectric elements. The ultrasonic signal cable 67 passes through the ultrasonic signal cable channel and is connected to the ultrasonic connector 68. Now, the ultrasonic signal cable channel is an insertion hole that is located within the bending portion 70 of the insertion portion 62, the flexible portion 71, the operating unit 63, the universal cord 65, and the endoscope connector 66.

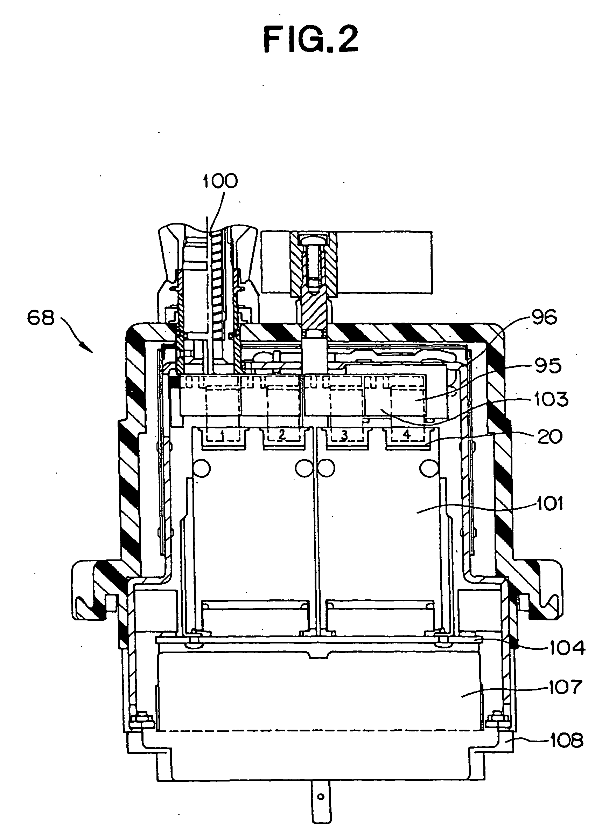

An ultrasonic signal cable connector device to be described below is provided on the distal end of the signal lines of each ultrasonic signal cable 67 for each group wherein the signal lines have been grouped together. This ultrasonic signal cable connector device is connected to the connecting connector 20 (see Fig. 2) of the ultrasonic connector 68.

Next, the configuration of the ultrasonic connector 68 will be described, with reference to Fig. 2. Fig. 2 is a cross-sectional diagram of an ultrasonic connector.

The ultrasonic connector 68 mainly comprises a metal frame 96 in an approximately rectangular box shape, a cable insertion hole 100, multiple connector receptacles 20, four to be specific, an insulating sheet 103, multiple matching boards 101, two to be specific, a base board 104, a metal board 107, and a connector unit 108. The cable insertion hole 100 is an insertion hole for inserting the ultrasonic signal cable 67 into the metal frame 96. Multiple ultrasonic signal cable connector devices to be described below, which are provided on the distal end of the ultrasonic signal cable 67 that is inserted from the cable insertion hole 100, are attached to the connector receptacle 20. The ultrasonic signal cable connector devices are each attached to connecting connectors, and in the moment wherein they are connected, the insulating sheet 103 is provided between the metal frame 96 and the ultrasonic signal cable connector device. In other words, the insulating sheet 103 insulates the metal frame 96 and the ultrasonic signal cable connector device in the moment that the ultrasonic signal cable connector device is connected to the connector receptacle 20. The matching boards 101 are electrically connected to the multiple connector receptacles 20. The matching boards 101 are boards wherein matching circuits are formed. The matching circuits perform the matching between the ultrasonic measuring equipment and the electronic scanning ultrasonic transducer that is connected to the ultrasonic signal cable 67. The base board 104 is connected to the multiple matching boards 101. Further, the base board 104 has multiple connector pins for the purpose of connecting to the ultrasonic measuring equipment. The metal board 107 covers the area of the connector pin of the base board 104, and is fixed to the metal frame 96. In the moment that the ultrasonic connector 68 is connected to the ultrasonic measuring equipment, the connector unit 108 has the metal frame 96 and the metal board 107 to be at the same electric potential as the reference potential of the ultrasonic measuring equipment.

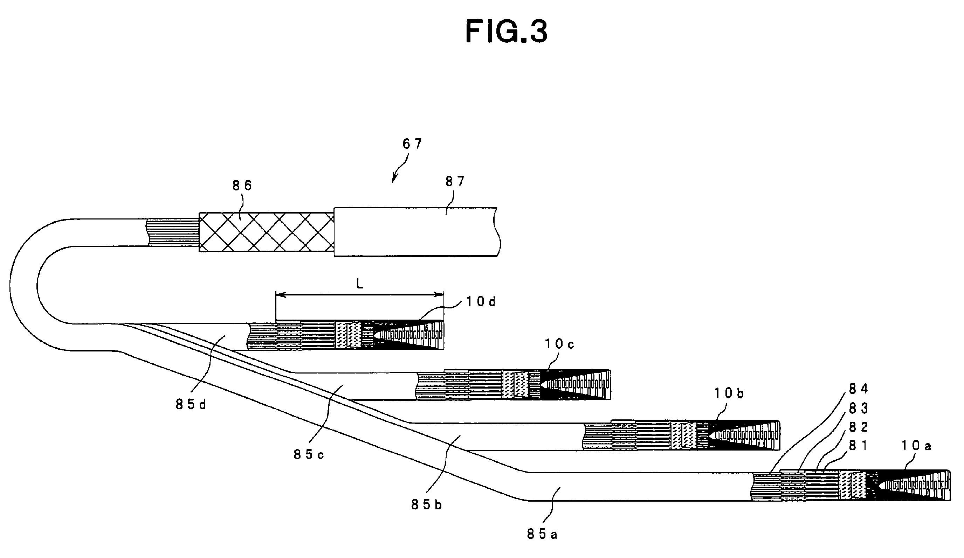

The configuration of the ultrasonic signal cable 67 that is connected to the ultrasonic connector 68 will be described with Fig. 3. The ultrasonic signal cable 67 comprises an overall insulator covering 87, multiple coaxial lines 85a through 85d, and an overall shield 86 that bundles the coaxial lines 85a through 85d. Multiple signal core lines 81 are located within the coaxial lines 85a through 85d.

The multiple signal core lines 81 are each covered with an insulator layer 82. A shield 83 surrounds each of the insulator layers 82. An insulator covering layer 84 surrounds and covers the shields 83. In other words, inside of the coaxial lines 85a through 85d, the signal core lines 81 that are covered by the insulator layer 82, the shield 83 and the insulator covering layer 84 are grouped together with several lines in a unit.

On the distal ends of the multiple coaxial lines 85a through 85d, ultrasonic signal cable connector devices 10a through 10d that are connecting boards are each connected (hereafter, the ultrasonic signal cable connector devices 10a through 10d may be individually or collectively referred to as "ultrasonic signal cable connector device 10"). The ultrasonic signal cable connector devices 10a through 10d are connected, in order, along the length of the ultrasonic signal cable 67 on the distal ends of the coaxial lines 85a through 85d, each set apart only the length L of the ultrasonic signal cable connector device 10. In other words, the ultrasonic signal cable connector devices 10a through 10d are located in a perpendicular direction to the length of the ultrasonic signal cable 67, so as not to overlap the others.

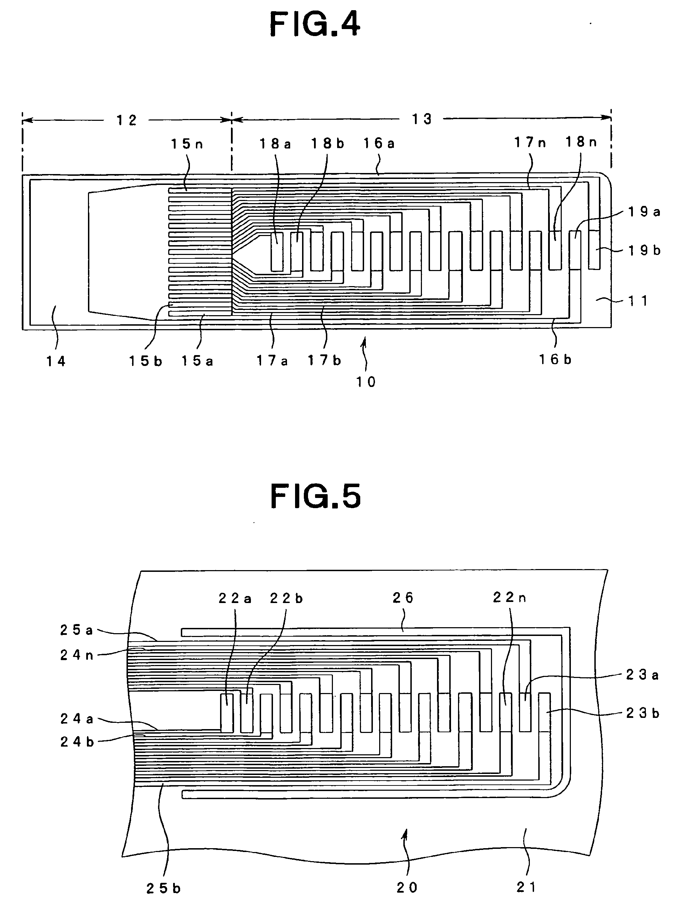

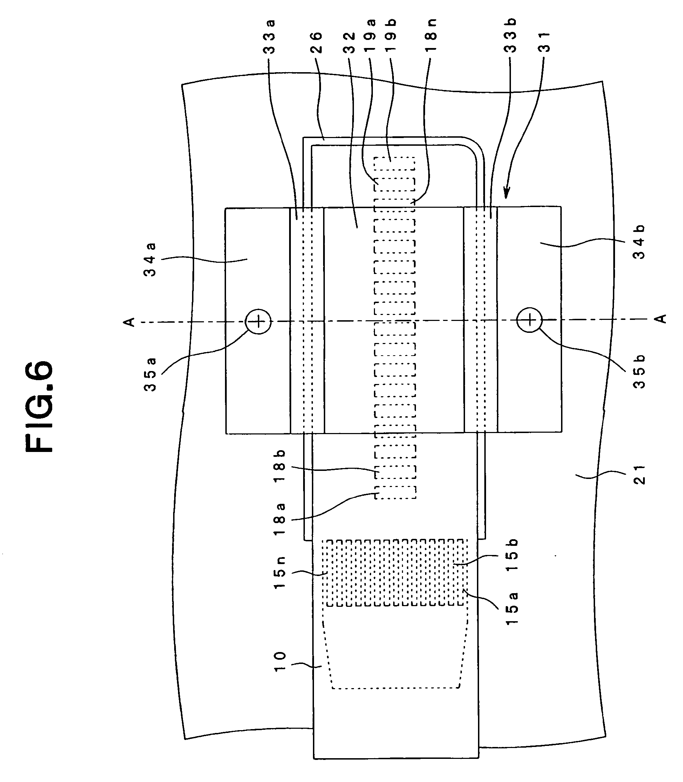

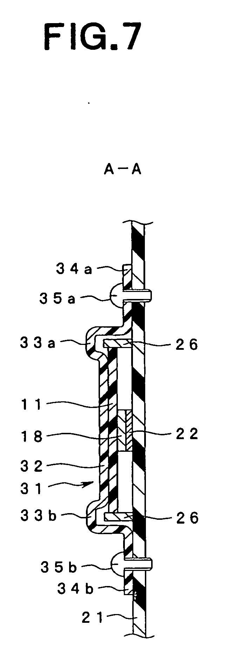

The ultrasonic signal cable connector device will be described below with reference to Figs. 4 through 6. Fig. 4 is a front view illustrating the configuration of the first embodiment of an ultrasonic signal cable connector device relating to the present invention, Fig. 5 is a back view illustrating the configuration of a connecting board of a connector receptacle provided on the ultrasonic connector of the first embodiment of the ultrasonic signal cable connector device relating to of the present invention, Fig. 6 is a plan view illustrating the attached state of the ultrasonic signal cable connector device to the ultrasonic connector relating to the present invention, and Fig. 7 is a sectional view illustrating the attached state of the ultrasonic signal cable connector device to the cable connector relating to the present invention.

As illustrated in Fig. 4, the ultrasonic signal cable connector device (hereafter referred to as "cable connector") 10 relating to the first embodiment of the present invention is a wiring board 11 wherein connection lands or wiring patterns are formed on the face of the insulation base board.

The wiring board 11 is formed in a rectangular shape, wherein the length of at least the short side is formed to be equal to or less than the external diameter of the ultrasonic signal cable 67 that is passed through the ultrasonic signal cable channel of the ultrasonic endoscope 61, or less than the internal diameter of the ultrasonic signal cable channel. Now, the wiring board 11 can be either a hard board or a soft flexible board.

The wiring board 11 is divided into a cable connecting portion 12 that connects the ultrasonic signal cable 67 lengthwise, and a connector connecting unit 13 that connects the ultrasonic connector 68. Multiple signal line connecting lands 15a through 15n and a grounding land 14 are provided on the cable connecting portion 12.

The signal line connecting lands 15a through 15n are located in a parallel line in the lengthwise direction of the wiring board 11, and are electrically connected to the multiple signal core lines 81 of the coaxial lines 85a through 85d of the ultrasonic signal cable 67 by means of soldering.

The grounding land 14 is electrically connected to the multiple shields 83 that are covering the multiple signal core lines 81 and the insulator layers 82 by means of soldering.

The connector connecting unit 13 provides multiple grounding patterns, two to be specific, 16a and 16b, multiple wiring patterns 17a through 17n, multiple contact piece lands 18a through 18n, and multiple grounding contact piece lands, two to be specific, 19a and 19b.

The multiple contact piece lands 18a through 18n are formed wherein each is approximately the same shape and size, and are located in a vertical row with approximately equal spacing therebetween, along the lengthwise direction of the central portion of the wiring board 11.

The multiple grounding contact piece lands 19a and 19b are formed wherein each is approximately the same shape and size as the contact piece lands 18a through 18n, and are located in a vertical row on the distal end side of the contact piece lands 18a through 18n (to the right side in Fig. 3), with approximately equal spacing between.

The grounding patterns 16a and 16b extend from the ends of both sides of the grounding lands 14 of the cable connecting portion 12, along the length of the surface of the wiring board 11, and are each connected to the corresponding grounding contact piece lands 19a and 19b.

The wiring patterns 17a through 17n extend from the signal line connecting lands 15a through 15n of the cable connecting portion 12, along the length of the surface of the wiring board 11, and are each connected to the corresponding contact piece lands 18a through 18n.

In other words, when the multiple signal core lines 81 of the ultrasonic signal cable 67 are connected by soldering to each of the signal line connecting lands 15a through 15n of the cable connecting portion 12, connection thereof is made electrically to the contact piece lands 18a through 18n via the wiring patterns 17a through 17n of the connector connection unit 13.

Further, when each of the shields 83 of the signal core lines 81 are connected by soldering to the grounding land 14 of the cable connecting portion 12, connection thereof is made electrically to the grounding contact piece lands 19a and 19b via the grounding patterns 16a and 16b of the connector connection unit 13.

The cable connector 10 thus configured is connected to each of the coaxial lines 85a through 85d on the ultrasonic signal cable 67.

As illustrated in Fig. 5, the above-described connector receptacle 20 has on the surface of the wiring board 21 multiple contact piece lands 18a through 18n provided on the connector connecting unit 13 of the cable connector 10, and multiple contact point lands 22a through 22n wherein each is approximately the same shape and size as the grounding contact piece lands 19a and 19b, with approximately equal spacing between, and multiple grounding contact point lands, two to be specific, 23a and 23b.

Multiple connecting patterns 24a through 24n and multiple ground patterns, two to be specific, 25a and 26b, each extend from the contact point lands 22a through 22n and the grounding contact point lands 23a and 23b, and are connected to the matching board 101.

In other words, the multiple contact point lands 22a through 22n and the two grounding contact point lands 23a and 23b that are provided on the surface of the wiring board 21 of the connector receptacle 20 are formed in a shape and size so as to overlap and make contact with the contact piece lands 18a through 18n and the grounding contact piece lands 19a and 19b respectively of the connector connecting unit 13 of the cable connector 10.

A connector attaching frame 26 that surrounds three sides of the area formed by the contact point lands 22a through 22n, the grounding contact point lands 23a and 23b, the wiring patterns 24a through 24n, and the grounding patterns 25a and 25b, is provided on the surface of the wiring board 21 of the connector receptacle 20. The connector attaching frame 26 is approximately the same shape as the wiring board 11 of the connector connecting unit 13 of the cable connector 10, and is a member for positioning of the connector connecting unit 13 in the moment that the connector connecting unit 13 is overlapped with the connector receptacle 20.

The method for connecting the cable connector 10 and the connector receptacle 20 will be described with reference to Fig. 6. The connector connecting unit 13 of the cable connector 10 is attached inside the connector attaching frame 26. When the connector connecting unit 13 is attached to the connector attaching frame 26, the contact piece lands 18a through 18n and the grounding contact piece lands 19a and 19b of the connector connecting unit 95 (see Fig. 2) of the cable connector 10 make contact with the contact point lands 22a through 22n and the ground contact point lands 23a and 23b of the connector receptacle 20, respectively.

The cable connector 10 that is attached to the connector attaching frame 26 is pressed down and fixed by a pressing fixation piece 31 of the connector receptacle 20. The pressing fixation piece 31 comprises a pressing face 32 that makes contact with at least the back face of the connector connecting unit 13 (see Fig. 4) of the cable connector 10, multiple recessed portions, two to be specific, 33a and 33b that give elasticity to the pressing face 32 as well as avoids contact with the connector attaching frame 26, multiple leg portions, two to be specific, 34a and 34b, extending from the recessed portions 33a and 33b that are located on the surface of the wiring board 21 of the connector receptacle 20, and multiple external thread screws, two to be specific, 35a and 35b, that fix the leg portions 34a and 34b to the wiring board 21 of the connector receptacle 20.

In other words, as illustrated in Fig. 7, after the connector connecting unit 13 of the cable connector 10 is attached to the connector attaching frame 26 of the connector receptacle 20, the pressing fixation piece 31 is placed on the back face of the cable connector 10, and fixes the leg portions 34a and 34b of the pressing fixation piece 31 with the two external thread screws 35a and 35b. Thus, in order for the pressing face 32 of the pressing fixation piece 31 to press the cable connector 10, the contact piece lands 17a through 17n and the grounding contact piece lands 18a and 18b of the connector connecting unit 13 of the cable connector 10 are pressed on to the corresponding contact point lands 22a through 22n and the grounding contact point lands 23 and 23b respectively, and each makes contact.

As described above, the cable connector 10 has a rectangular shape wiring board 11 wherein the width is less than at least the internal diameter of the ultrasonic signal cable channel of the endoscope insertion portion 62. The wiring board 11 of the cable connector 10 comprises two sections, the cable connecting portion 12 that has grounding lands 14 that are connected to the shields 83 and the multiple signal line connecting lands 15a through 15n that are positioned in a row to connect to the corresponding multiple signal core lines 81 of the ultrasonic signal cable 67, and the connector connecting unit 13 that has multiple contact piece lands 18a through 18n and grounding lands 19a and 19b that are positioned in a vertical row with approximately equal spacing in the center portion in the lengthwise direction of the wiring board 11 that are each connected to the signal line connecting lands 15a through 15n and the grounding land 14.

The cable connector 10 connects the contact piece lands 18a through 18n and the grounding lands 19a and 19b of the cable connector 10 to the corresponding contact point lands 22a through 22n and the grounding contact point lands 23a and 23b of the connector receptacle 20, by overlapping the connector receptacle 20 provided on the ultrasonic connector 68, and also by pressing and fixing the pressing fixation piece 31.

Now, an electroconductive member such as electroconductive rubber or the like may be placed on the contact surfaces between the lands 18a through 18n, 19a, 19b, 22a through 22n, 23a, and 23b, so that conductivity is ensured upon the contact piece lands 18a through 18n and the grounding lands 19a and 19b of the cable connector 10 each making contact with the corresponding contact point lands 22a through 22n and the grounding contact point lands 23a and 23b of the connector receptacle 20.

Consequently, with the ultrasonic signal cable connector device 10 of the present embodiment, soldering work to connect to the connecting connector of the ultrasonic connector 68 can be eliminated. Therefore, connection to the ultrasonic connector 68 is made easier and can be simplified.

### Second Embodiment

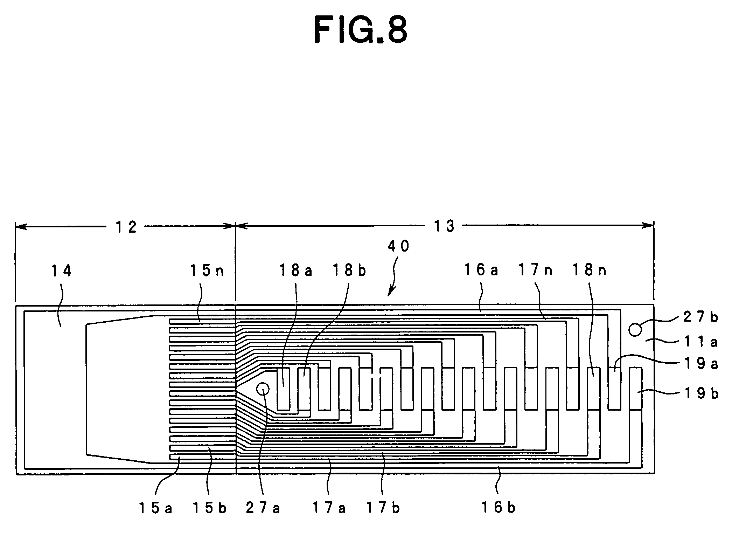

Next, an ultrasonic signal cable connector device relating to a second embodiment of the present invention will be described with reference to Figs. 8 through 16. Now, only the points of the present embodiment that differ from the configuration of the first embodiment will be described; configurations which are the same will be denoted with the same reference numerals and description thereof will be omitted.

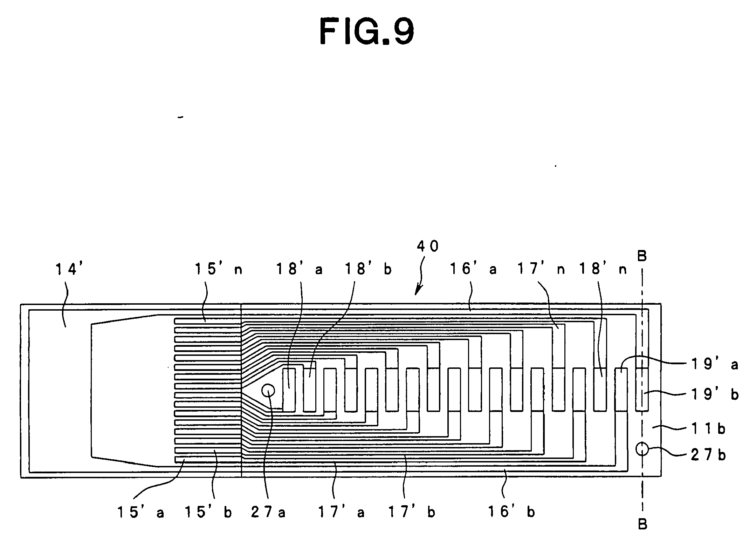

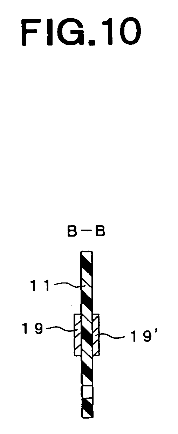

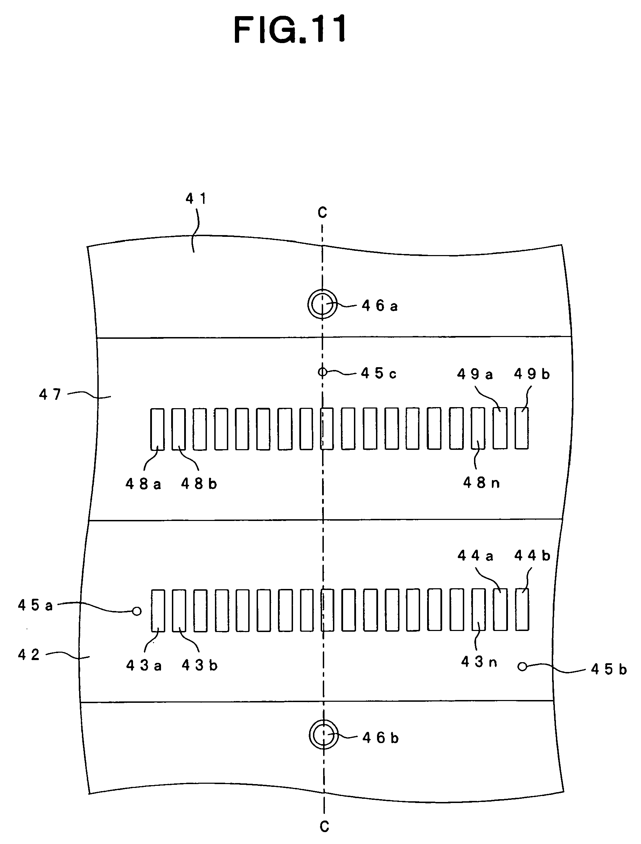

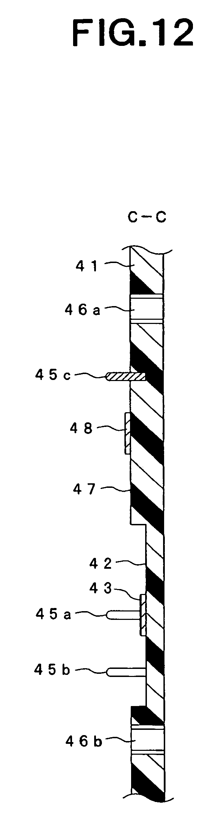

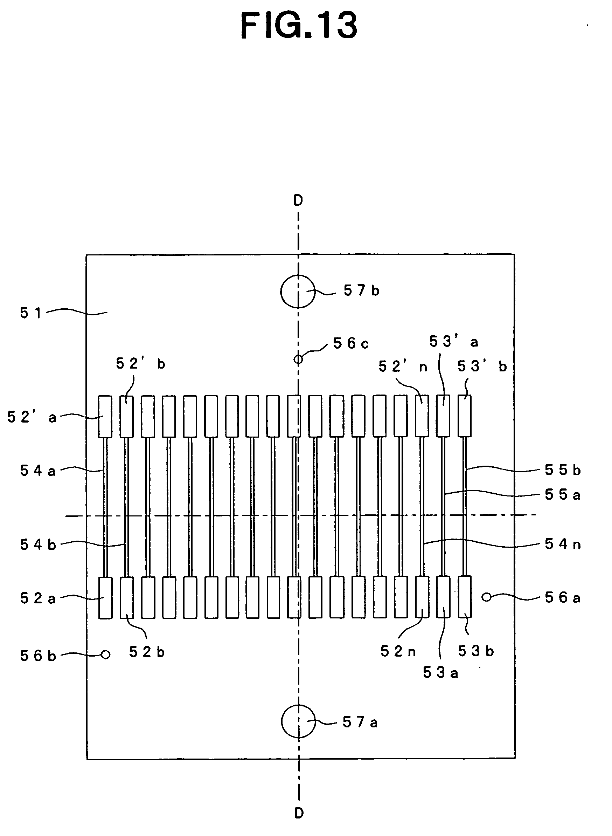

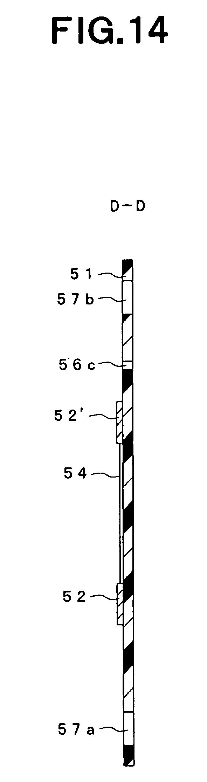

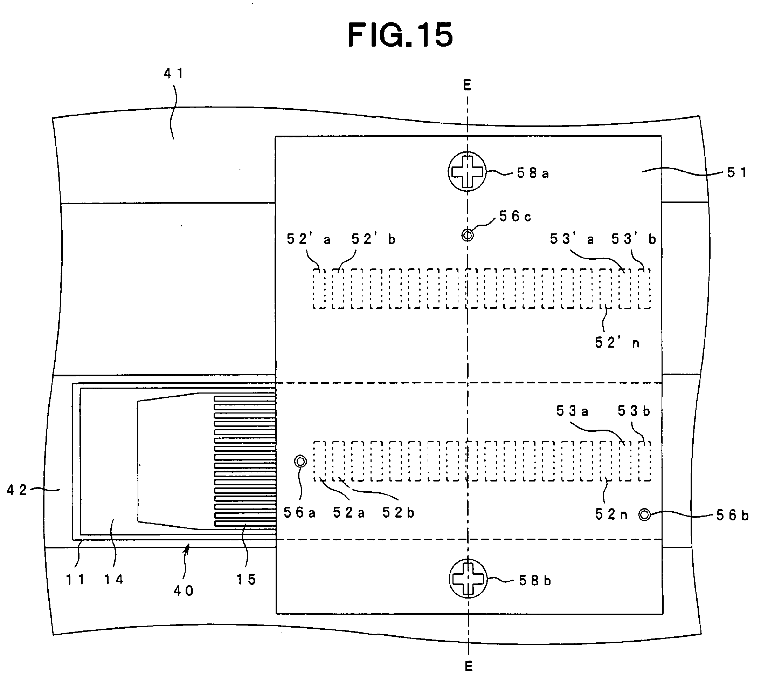

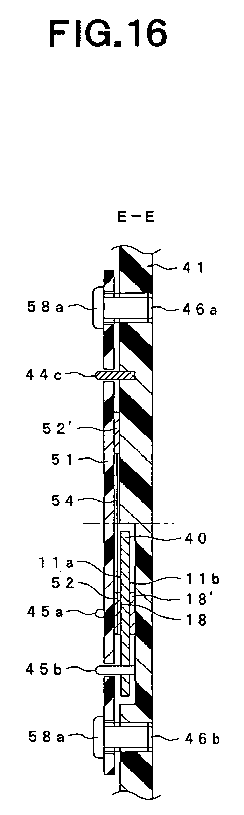

Fig. 8 is a plan view of the ultrasonic signal cable connector device, Fig. 9 is a back view of the ultrasonic signal cable connector device, Fig. 10 is a cross-sectional view of the ultrasonic signal cable connector device, Fig. 11 is a plan view illustrating the configuration of the connector receptacle wherein the ultrasonic signal cable connector of the ultrasonic signal cable connector device is attached, and Fig. 12 is a cross-sectional view illustrating the configuration of the connector receptacle wherein the ultrasonic signal cable connector of the ultrasonic signal cable connector device is attached. Fig. 13 is a plan view illustrating the configuration of a connecting board for connecting the ultrasonic signal cable connector of the ultrasonic signal cable connector device and the connector receptacle, Fig. 14 is a cross-sectional view illustrating the configuration of a connecting board for connecting the ultrasonic signal cable connector of the ultrasonic signal cable connector device and the connector receptacle, Fig. 15 is a plan view illustrating the attached state of the ultrasonic signal cable connector of the ultrasonic signal cable connector device and the connector receptacle, and Fig. 16 is a cross-sectional view illustrating the attached state of the ultrasonic signal cable connector of the ultrasonic signal cable connector device and the connector receptacle.

The above-described cable connector 10 according to the first embodiment of the present invention has a grounding land 14, a signal line connecting land 15, a grounding pattern 16, a wiring pattern 17, an contact piece land 18, and a grounding contact piece land 19 on one surface of the wiring board 11.

On the other hand, the cable connector 40 of the present embodiment has two grounding lands 14 and 14', one on each face, multiple signal line connecting lands 15a through 15n and 15'a through 15'n, a total of four grounding patterns 16a, 16b, 16'a, 16'b, two on each face, multiple wiring patterns 17a through 17n and 17'a through 17'n, multiple contact piece lands 18a through 18n and 18'a through 18'n, and a total of four grounding contact piece lands 19a, 19b, 19'a, 19'b, two on each face, on both faces 11a and 11b of the wiring board 11, as illustrated in Figs. 8 through 10. Hereafter, the description will refer to only the numeral of the reference numeral for each land and each pattern (for example, the signal line connecting lands 15a through 15n and 15'a through 15'n will be referred to as signal line connecting lands 15 and 15').

The grounding lands 14 and 14', the signal line connecting lands 15 and 15', the grounding patterns 16 and 16', the wiring pattern 17 and 17', the contact piece lands 18 and 18', and the grounding contact piece lands 19 and 19' that are provided on both faces 11a and 11b of the wiring board 11 are formed with approximately the same shape and size for each land and pattern. Now, the two faces 11a and 11b of the wiring board 11 may be referred to as the front face 11a and back face 11b in the description below.

Thus, the two faces 11a and 11b of the wiring board 11 each has two grounding lands 14 and 14', one on each face, multiple signal line connecting lands 15 and 15', a total of four grounding patterns 16 and 16', two on each face, multiple wiring patterns 17 and 17', multiple contact piece lands 18 and 18', and a total of four grounding contact piece lands 19 and 19', two on each face, and thus the number of signal lines on the ultrasonic signal cable 67 connected to the cable connector 40 can be approximately doubled. In other words, the two coaxial lines 85a and 85b of the ultrasonic signal cable 67 described with reference to Fig. 3 can be grouped together as one, and further, the multiple signal core lines 81 within the coaxial lines 85a and 85b can be connected to one cable connector 40.

Now, the connector connecting unit 13 of the cable connector 40 has a first positioning hole 27a near the center of the cable connecting portion 12, and a second positioning hole 27b near one corner of the distal end side of the connector connecting unit 13.

First and second positioning shafts 45a and 45b of the connector receptacle 41 that is the wiring board to be described below, which is the connecting receiving unit 20 in the first embodiment, provided on the connecting connector 102 of the ultrasonic connector 68, are passed through the first and second positioning holes 27a and 27b.

Next, the connector receptacle 41 provided on the connecting connector 102 of the ultrasonic connector 68 that corresponds to the cable connector 40 wherein the ultrasonic signal cable 67 is capable of being connected will be described with reference to Figs. 11 and 12.

On one face of the connector receptacle 41 a rectangular recessed portion 42 wherein the cable connector 40 attaches is formed. The recessed portion 42 is formed to have approximately the same thickness as the thickness of the wiring board 11 of the cable connector 40, and is a recessed portion formed in approximately the same shape as the wiring board 11. In the center portion in the lengthwise direction of the recessed portion 42 bottom face is provided the multiple first contact point lands 43a through 43n and multiple first grounding contact points lands, two to be specific, 44a and 44b that are positioned with approximately equal spacing and formed to have approximately similar shape and size as the corresponding multiple contact piece lands 18'a through 18'n and the two grounding contact piece lands 19'a and 19'b provided on the back face 11b of the wiring board 11 of the cable connector 40.

A first positioning shaft 45a that is passed through the first positioning hole 27a of the cable connector 40, as seen in Fig. 11 near the left side in the drawing, of the first contact point lands 43a through 43n, is provided on the recessed portion 42 so as to protrude in the perpendicular direction from the bottom face thereof. Further, a second positioning shaft 45b that is passed through the second positioning hole 27b of the cable connector 40, as seen in Fig. 11 to the lower side, of the first contact point land 44b, is provided on the recessed portion 42 so as to protrude in the perpendicular direction from the front face thereof.

In other words, the two, first and second, positioning shafts 45a and 45b are passed through the two, first and second, positioning holes 27a and 27b of the wiring board 11 from the back face side of the cable connector 40, and the wiring board 11 is attached to the recessed portion 42. Thus, the contact piece lands 18'a through 18'n and the grounding contact piece lands 19'a and 19'b of the back face 11b of the wiring board 11 each makes contact with the corresponding first contact point lands 43a through 43n and the first grounding contact point lands 44a and 44b of the recessed portion 42.

Now, a wiring pattern and a grounding pattern for the purpose of connecting to the matching board 101 each extend from the first contact point lands 43a through 43n and the first grounding contact point lands 44a and 44b of the recessed portion 42, although this is not illustrated.

Further, multiple second contact point lands 48a through 48n and multiple second grounding contact point lands, two to be specific, 49a and 49b are formed on the plane portion 47 adjacent to the recessed portion 42 of the connector receptacle 41. The second contact point lands 48a through 48n and the grounding contact point lands 49a and 49b are formed to have approximately the same shape and size as the corresponding first contact point lands 43a through 43n and grounding contact point lands 44a and 44b, and are positioned on the surface of the plane portion 47 with approximately equal spacing.

A third positioning shaft 45c is provided near the side in the center section in the lengthwise direction (the upper part of Fig. 11) of the second contact point lands 48a through 48n of the plane portion 47. Now, two screw holes 46a and 46b, wherein the external thread screws are screwed in to attach and fix the connecting board 51 to be described below, are provided near one side corresponding to the lengthwise direction (the vertical direction of Fig. 11) of the recessed portion 42 and the plane portion 47 of the connector receptacle 41.

Now, a wiring pattern and a grounding pattern for the purpose of connecting to the matching board 101 each extend from the second contact point lands 48a through 48n and the second grounding contact point lands 49a and 49b of the plane portion 47, although this is not illustrated.

Next, the connecting board 51 will be described with reference to Figs. 13 and 14. On one face of the connecting board 51, multiple third contact point lands 52a through 52n and multiple third grounding contact point lands, two to be specific, 53a and 53b are provided. The multiple third contact point lands 52a through 52n and the two grounding contact point lands 53a and 53b are formed in approximately the same shape and size so as to make contact with the respective multiple contact piece lands 18a through 18n and the two grounding contact piece lands 19a and 19b provided on the front face 11a of the wiring board 11 of the corresponding cable connector 40, and are positioned with approximately equal spacing, and are located after the position is determined on one face of the connecting board 51.

Further, multiple fourth contact point lands 52'a through 52'n and multiple fourth grounding contact point lands, two to be specific, 53'a and 53'b are provided on one side of the connecting board 51 so as to line up with the third contact point lands 52a through 52n and the third grounding contact point lands 53a and 53b. The fourth contact point lands 52'a through 52'n and the fourth grounding contact point lands 53'a and 53'b are formed in approximately the same shape and size so as to make contact with the corresponding second contact point lands 48a through 48n and the second grounding contact point lands 49a and 49b of the connector receptacle 41, and are also positioned with approximately equal spacing and determined where to be positioned on the face of the connecting board 51.

The third contact point lands 52a through 52n and the third grounding contact point lands 53a and 53b of the connecting board 51 are electrically connected to the corresponding fourth contact point lands 52'a through 52'n and the fourth grounding contact point lands 53'a and 53'b by the wiring patterns 54a through 54n or the grounding patterns 55a and 55b.

Further, first through third positioning holes 56a through 56c provided in the positions corresponding to the first through third positioning shafts 45a through 45c provided on the connector receptacle 41, and screw openings 57a and 57b provided in the positions corresponding to the attaching screw holes 46a and 46b of the connector receptacle 41, are bored in the connecting board 51.

The connection of the cable connector 40, the connector receptacle 41, and the connecting board 51, with such a configuration, will be described with reference to Figs. 15 and 16.

The multiple signal core lines 81 (see Fig. 3) of the ultrasonic signal cable 67 are connected to the signal line connecting lands 15a through 15n, the grounding land 14, the signal line connecting lands 15'a through 15'n, and the grounding land 14' provided on both faces 11a and 11b of the wiring board 11 of the cable connector 40.

For example, the signal core lines 81 and the shields 83 of the coaxial lines 85a are each soldered to the corresponding signal line connecting lands 15a through 15n and the grounding contact piece land 14 on the front face 11a of the wiring board 11, and further, the signal core lines 81 and the shields 83 of the coaxial lines 85b are each soldered to the corresponding signal line connecting lands 15'a through 15'n and the grounding contact piece land 14' on the back face 11b of the wiring board 11, and are thus electrically connected.

The cable connector 40 that is electrically connected to the ultrasonic signal cable 67 is inserted from the distal end side of the endoscope insertion unit 71 (see Fig. 1), and passes through to extend to the ultrasonic connector 68. The connector receptacle 41 is provided on the connecting connector 102 of the ultrasonic connector 68, and is attached to the recessed portion 42 of the connector receptacle 41 from the back face 11b side of the wiring board 11 of the cable connector 40.

At this time, the first and second positioning shafts 45a and 45b provided on the recessed portion 42 are passed through the first and second positioning holes 27a and 27b provided on the cable connector 40, and the cable connector 40 is attached to the connector receptacle 41. When the position of the cable connector 40 is determined by the first and second positioning shafts 45a and 45b and attached to the recessed portion 42 of the connector receptacle 41, the contact piece lands 18'a through 18'n and the grounding contact piece lands 19'a and 19'b provided on the back face 11b side of the wiring board 11 electrically connect to the corresponding contact point lands 43a through 43n and the grounding contact point lands 44a and 44b of the recessed portion 42.

When the cable connector 40 is attached to the recessed portion 42 of the connector receptacle 41, the first through third positioning holes 56a through 56c of the connecting board 51 are each inserted into the first through third positioning shafts 45a through 45c of the connector receptacle 41. Then, one face of the connecting board 51 is positioned on the connector receptacle 41 so as to face the plane portion 47 of the connector receptacle 41 and the front face 11a of the wiring board 11 of the cable connector 40 that is attached to the recessed portion 42.

In other words, when the connecting board 51 is positioned on the connector receptacle 41, the third contact point lands 52a through 52n and the grounding contact point lands 53a and 53b make contact with the corresponding contact piece lands 18a through 18n and the grounding contact piece lands 19a and 19b provided on the front face 11a of the wiring board 11.

Further, the fourth contact point lands 52'a through 52'n and the grounding contact point lands 53'a and 53'b on the connecting board 51 make contact with the corresponding second contact point lands 48a through 48n and the grounding contact point lands 49a and 49b provided on the plane portion 47 of the connector receptacle 41. In this state, the connecting board 51 is fixed to the connector receptacle 41 by means of the external thread screws 58a and 58b passing through the screw openings 57a and 57b, and screwing the connecting board 51 in place to the attaching screw holes 46a and 46b of the connector receptacle 41.

Thus, the contact piece lands 18a through 18n and 18'a through 18'n and the grounding lands 19a, 19b, 19'a, and 19'b provided on the two sides 11a and 11b of the wiring board 11 of the cable connector 40 are electrically connected to the matching board 101 via the connector receptacle 41 and the connecting board 51.

Accordingly, the cable connector 40 can be made with a smaller sideways width because of the multiple signal core lines 81 of the ultrasonic signal cable 67 being capable of connecting to both sides. Therefore, insertion of the endoscope insertion portion 62 into the ultrasonic signal cable channel can be greatly improved. Further, the endoscope insertion portion 62 itself can have a smaller diameter because the ultrasonic signal cable channel of the endoscope insertion portion 62 has a smaller diameter.

Further, the cable connector 40 can have fewer connectors to the ultrasonic signal cable 67 because the many signal core lines 81 of the ultrasonic signal cable 67 are connected to the two faces 11a and 11b of the wiring board 11 of the cable connector 40, and the insertion operation into the ultrasonic signal cable channel becomes easier.

Further, the cable connector 40 is easily connected to the ultrasonic connector 68 by being sandwiched between the connector receptacle 41 and the connecting board 51, wherein the connector receptacle 41 overlaps and attaches the cable connector 40 with the ultrasonic connector 68. In particular, since no soldering work is necessary for the connection between the cable connector 40 and the ultrasonic connector 68, attaching the cable connector 40 to the ultrasonic connector 68 can be performed more efficiently.

### Third Embodiment

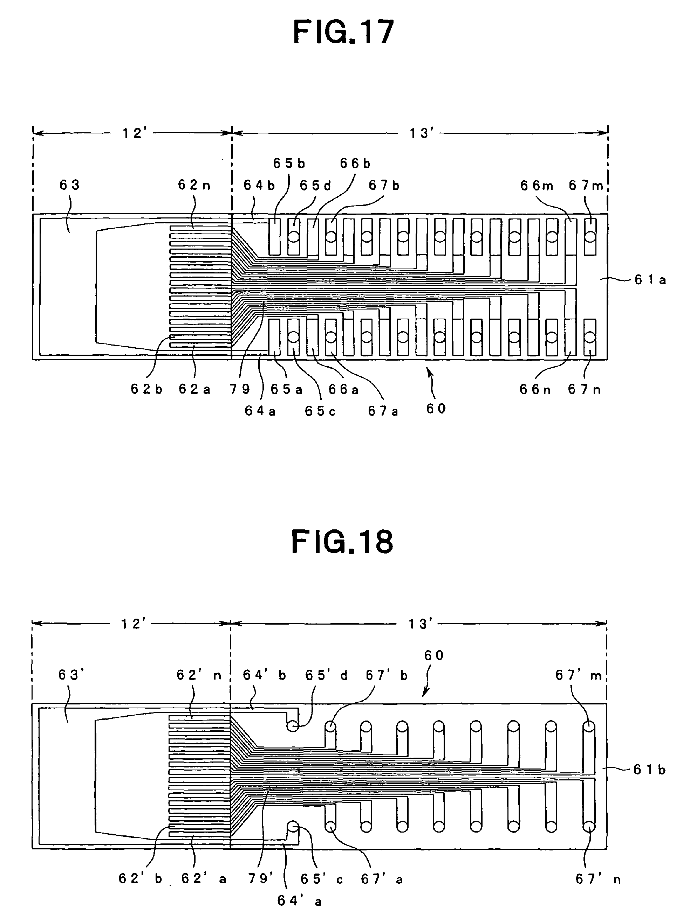

Next, the ultrasonic signal cable connector device relating to the present invention will be described with reference to Figs. 17 and 18.

Fig. 17 is a front view illustrating the configuration of the ultrasonic signal cable connector device relating to the third embodiment of the present invention, and Fig. 18 is a back view illustrating the configuration of the ultrasonic signal cable connector device according to the third embodiment of the present invention.

The cable connector 60 of the present embodiment is provided with multiple signal line connecting lands 62a through 62n and 62'a through 62'n and two grounding lands, one on each face, 63 and 63' that are electrically connected by soldering the shield 83 and the signal core line 81 of the ultrasonic signal cable 67 to both the front and back faces of the cable connecting portion 12' of the wiring board 61 that is formed in a rectangular shape, similar to the above-described second embodiment.

The connector connecting unit 13' of one face (hereafter may be referred to as "front face") 61a of the wiring board 61 of the cable connector 60 is provided with multiple grounding patterns, two to be specific, 64a and 64b that extend from both ends of the connecting land 63 of the cable connecting portion 12', and multiple wiring patterns 79 that each extends from the multiple signal line connecting lands 62a through 62n, and two grounding contact piece lands 65a and 65b that each connects to the grounding patterns 64a and 64b, and multiple contact piece lands 66a through 66n that each connects to the wiring patterns 79, and multiple grounding contact piece lands that have a through hole (hereafter referred to as "grounding contact piece land with through hole") 65c and 65d, and multiple contact piece lands that have a through hole (hereafter referred to as "contact piece land with through hole") 67a through 67n.

The wiring patterns 79 are formed with predetermined spacing that is approximately equal to the right and left thereof, towards the center shaft in the lengthwise direction of the wiring board 61 that is in a rectangular shape on the front face 61a of the wiring board 61. The grounding contact piece lands 65a and 65b and the contact piece lands 66a through 66n are formed in approximately the same shape and size, and positioned with equal spacing, along both long edges of the rectangular shaped wiring board 61 on the front face 61a of the wiring board 61.

The two grounding contact piece lands with through holes 65c and 65d, and the multiple contact piece lands with through holes 67a through 67n, are formed in approximately the same shape as the two grounding lands 64a and 64b, and the multiple contact piece lands 66a through 66n, and are each positioned between the grounding lands 64a and 64b, and the contact piece lands 66a through 66n.

As illustrated in Fig. 18, the connector connecting unit 13' of the other face (hereafter may be referred to as "back face") 61b of the wiring board 61 of the cable connector 60 is provided with multiple grounding patterns, two to be specific, 64'a and 64'b that extend from both ends of the connecting land 63' of the cable connecting portion 12', and multiple wiring patterns 79' that each extends from the signal line connecting lands 62'a through 62'n, and multiple grounding through holes, two to be specific, 65'c and 65'd that each connects to the two grounding patterns 64'a and 64'b, and multiple through holes 67'a through 67'n that are each connected to the multiple wiring patterns 79'.

The wiring patterns 79' are formed with predetermined spacing that is approximately equal to the right and left thereof, towards the center shaft in the lengthwise direction of the wiring board 61a that is in a rectangular shape on the back face 61b of the wiring board 61. The grounding through holes 65'c and 65'd and the through holes 67'a through 67'n are each electrically connected to the grounding contact piece lands with through holes 65c and 65d and the contact piece lands with through holes 67a through 67n that are provided on the front face 61a of the wiring board 61.

In other words, the cable connector 60 is arranged such that the multiple signal line connecting lands 62a through 62n and 62'a through 62'n and the two grounding contact piece lands 63 and 63' formed on the front and back faces 61a and 61b of the cable connecting portion 12' of the wiring board 61 can connect to the corresponding multiple signal core lines 81 and the multiple shields 83 of the ultrasonic signal cable 67. The multiple signal core lines 81 and the multiple shields 83 of the ultrasonic signal cable 67 connected to the front face 61a of the wiring board 61 of the cable connector 60 are connected to the corresponding grounding contact piece lands 65a and 65b and the connecting contact piece lands 66a through 66n via the grounding patterns 64a and 64b and the wiring patterns 65a through 65n.

Further, the multiple signal core lines 81 and the multiple shields 83 of the ultrasonic signal cable 67 connected to the back face 61b of the wiring board 61 are connected to the corresponding grounding through holes 65'c and 65'd and the through holes 67'a through 67'n via the grounding patterns 64'a and 64'b and the wiring patterns 79'.

In other words, the multiple signal core lines 81 and the multiple shields 83 of the ultrasonic signal cable 67 connected to the back face 61b of the wiring board 61 are electrically connected to the corresponding grounding contact piece lands with through hole 65c and 65d and the contact piece lands with through hole 66a through 66n provided on the front face 61a of the wiring board 61, via the grounding through holes 65'c and 65'd and the through holes 67'a through 67'n.

Therefore, the cable connector 60 enables the cable connecting portion 12' to connect the signal core lines 81 and the shields 83 of the ultrasonic signal cable 67 to the two faces 61a and 61b of the wiring board 61, and the multiple contact piece lands 65a through 65d and 66a through 66n and 67a through 67n that are connected to the ultrasonic connector 68 are provided on one face. The multiple signal core lines 81 and the shields 83 connected to the back face 61b of the wiring board 61 are electrically connected to the corresponding contact piece lands 65c and 65d and 67a through 67n provided on the front face 61a of the wiring board 61, via each of the through holes 65'c, 65'd, and 67'a through 67'n.

Note that, contact point lands are formed on the connector receptacle 41 for connecting the above-described cable connector 60 to the ultrasonic connector 68 which can make connection facing the corresponding grounding contact piece lands 65a through 65d, the contact piece lands 66a through 66n, and the contact piece lands with through hole 67a through 67n of the connector connecting unit 13' of the cable connector 60. Thus, the cable connector 60 easily connects to the ultrasonic connector 68.

As described above, the ultrasonic signal cable connector device according to the present invention can be formed so as to be capable of passing through the ultrasonic cable channel of an endoscope and so forth, and can be connected to the ultrasonic connector without necessitating soldering work.

Having described the preferred embodiments of the invention referring to the accompanying drawings, it should be understood that the present invention is not limited to those precise embodiments and various changes and modifications thereof could be made by one skilled in the art without departing from the scope of the invention as defined in the appended claims.

## Claims

1. An ultrasonic signal cable connector device (10) to be used with
an ultrasonic endoscope device comprising
an ultrasonic endoscope (61) having an electronic scanning ultrasonic transducer (73) provided on a distal end (69) of an insertion portion to be inserted in a body cavity, for generating an ultrasonic wave to the body cavity tissue and receiving the ultrasonic wave reflected from the body cavity tissue so as to generate ultrasonic signals, and
ultrasonic measuring equipment for performing driving control of the ultrasonic transducer (73) of the ultrasonic endoscope (61) and generating ultrasonic image signals generated from the ultrasonic signal,
the ultrasonic signal cable connector device (10) comprising:
an ultrasonic signal cable (67) having a plurality of signal lines (81) adapted to be connected to the electrornic scanning ultrasonic transducer (73);
an ultrasonic connector (68) for connecting the ultrasonic signal cable (67) to the ultrasonic measuring equipment;
a rectangular wiring board (11) that has approximately the same or smaller width dimension as the external diameter of the ultrasonic signal cable (67);
a plurality of signal line connecting lands (15a...15n) arrayed in parallel on the surface of one of the short sides of the rectangular wiring board (11) electrically connecting the plurality of signal lines (81) of the ultrasonic signal cable (67);
a plurality of contact piece lands (18a...18n) arrayed in a column longitudinally on the surface of the rectangular wiring board (11) for electrically connecting a plurality of connecting connector pieces provided to the ultrasonic connector (68);
a plurality of wiring patterns (17a...17n) for connecting the plurality of signal line connecting lands (15a...15n) and contact piece lands (18a...18n) that are provided on the surface of the rectangular wiring board (11);
a connector receptacle (20) to which the rectangular wiring board (11) is fixed, the connector receptacle (20) being provided to the ultrasonic connector (68); and
a plurality of contact point lands (22a ... 22n) provided on a wiring board (21) of the connector receptacle (20), a plurality of connecting patterns (24a ... 24n) extending from the contact point lands for electrically connecting to a plurality of connector pins for connecting to the ultrasonic measuring equipment, the contact point lands being formed in shape and size so as to overlap the contact piece lands and being clamped to the contact piece lands.

2. An ultrasonic signal cable connector device (10) according to Claim 1, wherein the plurality of signal line connecting lands (15a...15n), contact piece lands (18a...18n), and wiring patterns (17a...17n) are provided on both the front (11a) and back surfaces (11b) of the rectangular wiring board (11).

3. An ultrasonic signal cable connector device (10) according to Claim 1, wherein the plurality of signal line connecting lands (15a...15n) are arrayed in parallel on the front (11a) and back surfaces (11b) of one of the short sides of the rectangular wiring board (11), the plurality of contact piece lands (18a ... 18n) are positioned in columns on both long sides on the surface of the rectangular wiring board (11), and the signal line connecting lands (15a...15n) provided on the back surface (11b) of the rectangular wiring board (11) are connected to the contact piece lands (18a...18n) via through holes (27a, 27b).

4. An ultrasonic signal cable connector device (10) according to Claim 2, the ultrasonic connector (68) comprising:
a connector receptacle (20) having
a plurality of first contact point lands (43a...43n) connected to the contact piece lands (18a...18n) provided on one face (11a) of the rectangular wiring board (11), and
a plurality of second contact point lands (48a...48n) disposed alongside the plurality of first contact point lands (43a...43n); and
a connecting board (51) having
a plurality of third contact point lands(52a...52n) connected to the contact piece lands (18a...18n) provided on the other face (11b) of the rectangular wiring board (11),
a plurality of fourth contact point lands(52'a...52'n) connected to the plurality of second contact point lands(48a...48n) of the connector receptacle (20), and
a wiring pattern (54a...54n) that connects the third (52a..:52n) and fourth contact point lands (52'a...52'n).

5. An ultrasonic endoscope (61) comprising:
an ultrasonic transducer (73) provided on a distal end (69) of an insertion portion to be inserted in a body cavity, for generating an ultrasonic wave to the tissue of the body cavity and receiving the ultrasonic wave reflected from the body cavity tissue so as to generate an ultrasonic signal;
an ultrasonic signal cable (67) having a plurality of signal lines (81) wherein one end is connected to a plurality of piezoelectric elements making up the ultrasonic transducer (73), and the other end is connected to an ultrasonic signal cable connector device (10); and
an ultrasonic connector (68) connected to the ultrasonic signal cable (67) and connected to ultrasonic measuring equipment;
the ultrasonic signal cable connector device (10) comprising:
a rectangular wiring board (11) that has approximately the same or smaller width dimension as the external diameter of the ultrasonic signal cable (67);
a plurality of signal line connecting lands (15a...15n) arrayed in parallel on the surface of one of the short sides of the rectangular wiring board (11) electrically connecting the plurality of signal lines (81) of the ultrasonic signal cable (67);
a plurality of contact piece lands (18a...18n) arrayed in a column longitudinally on the surface of the rectangular wiring board (11) for electrically connecting a plurality of connecting connector pieces provided to the ultrasonic connector (68); and
a plurality of wiring patterns (17a...17n) for connecting the plurality of signal line connecting lands (15a...15n) and contact piece lands (18a ... 18n) that are provided on the surface of the rectangular wiring board (11);
a connector receptacle (20) to which the rectangular wiring board (11) is fixed, the connector receptacle (20) being provided to the ultrasonic connector (68); and
a plurality of contact point lands (22a ... 22n) provided on a wriring oard (21) of the connector receptacle (20), a plurality of connecting patterns (24a ... 24n) extending from the contact point lands (22a...22n), for electrically connecting to a plurality of connector pins for connecting to the ultrasonic measuring equipment, the contact point lands (22a ... 22n) being formed in shape and size so as to overlap the contact piece lands and being clamped to the contact piece lands (15a ... 15n).

6. An ultrasonic endoscope (61) according to Claim 5, wherein the plurality of signal line connecting lands (15a...l5n), contact piece lands (18a...18n), and wiring patterns (17a...17n) are provided on both the front (11a) and back surfaces (11b) of the rectangular wiring board (11).

7. An ultrasonic endoscope (61) according to Claim 5, wherein the plurality of signal line connecting lands (15a...15n) are arrayed in parallel on the front (11a) and back surfaces (11b) of one of the short sides of the rectangular wiring board (11), the plurality of contact piece lands (18a...18n) are positioned in columns on both long sides of the rectagular wiring board (11), and the signal line connecting lands (15a...15n) provided on the back surface (11b) of the rectangular wiring board (11) are connected to the contact piece lands (18a...18n) via through holes (27a, 27b).

8. An ultrasonic endoscope (61) according to Claim 6, the ultrasonic connector (68) comprising:
a connector receptacle (20) having
a plurality of first contact point lands(43a...43n) connected to the contact piece lands(18a...18n) provided on one face (11a) of the rectagular wiring board (11), and
a plurality of second contact point lands (48a...48n) disposed alongside the plurality of first contact point lands(43a...43n); and
a connecting board (51) having
a plurality of third contact point lands(52a...52n) connected to the contact piece lands (18a...18n) provided on the other face (11b) of the rectangular wiring board (11),
a plurality of fourth contact point land (52'a...52'n) connected to the plurality, of second contact point lands (48a...48n) of the connector receptacle (20), and
a wiring pattern (54a...54n) that connects the third (52a...52n) and fourth contact point lands (52'a...52'n).

## Patentansprüche

1. Ultraschallsignal-Kabelverbindervorrichtung (10) zur Verwendung mit einer Ultraschall-Endoskopvorrichtung, mit:
einem Ultraschall-Endoskop (61) mit einem elektronischen Abtast-Ultraschallwandler (73), der am distalen Ende (69) eines Einführabschnitts vorgesehen ist, der in einen Körperhohlraum einzuführen ist, um eine Ultraschallwelle zum Gewebe des Körperhohlraums zu erzeugen und die vom Gewebe des Körperhohlraums reflektierte Ultraschallwelle zu empfangen, um Ultraschallsignale zu erzeugen, und
einer Ultraschall-Messausrüstung zum Ausführen der Ansteuerung des Ultraschallwandlers (73) des Ultraschall-Endoskops (61) und zum Erzeugen von Ultraschall-Bildsignalen, die aus dem Ultraschallsignal erzeugt werden,
wobei die Ultraschallsignal-Kabelverbindervorrichtung (10) aufweist:
ein Ultraschall-Signalkabel (67) mit einer Mehrzahl Signalleitungen (81), die zum Verbinden mit dem elektronischen Abtast-Ultraschallwandler (73) eingerichtet sind;
einen Ultraschallverbinder (68) zum Verbinden des Ultraschall-Signalkabels (67) mit der Ultraschall-Messausrüstung;
eine rechteckige Verdrahtungsplatine (11), die etwa die gleiche wie oder eine kleinere Breitenabmessung hat als der Außendurchmesser des Ultraschall-Signalkabels (67);
eine Mehrzahl Signalleitungs-Verbindungsstege (15a ... 15n), die parallel auf der Oberfläche einer der kurzen Seiten der rechteckigen Verdrahtungsplatine (11) angeordnet sind und die Mehrzahl Signalleitungen (81) des Ultraschall-Signalkabels (67) elektrisch verbinden;
eine Mehrzahl Kontaktstückstege (18a ... 18n), die in Form einer Spalte in Längsrichtung auf der Oberfläche der rechteckigen Verdrahtungsplatine (11) angeordnet sind, um eine Mehrzahl Anschluss-Verbinderstücke, die für den Ultraschallverbinder (68) vorgesehen sind, elektrisch zu verbinden;
eine Mehrzahl Verdrahtungsmuster (17a ... 17n) zum Verbinden der Mehrzahl Signalleitungs-Verbindungsstege (15a ... 15n) und Kontaktstückstege (18a ... 18n), die auf der Oberfläche der rechteckigen Verdrahtungsplatine (11) vorgesehen sind; eine Verbinderbuchse (20), an der die rechteckige Verdrahtungsplatine (11) befestigt ist, wobei die Verbinderbuchse (20) für den Ultraschallverbinder (68) vorgesehen ist; und
eine Mehrzahl Kontaktpunktstege (22a ... 22n), die auf einer Verdrahtungsplatine (21) der Verbinderbuchse (20) vorgesehen sind, eine Mehrzahl Verbindungsmuster (24a ... 24n), die sich von den Kontaktpunktstegen (22a ... 22n) aus erstrecken, um die Verbindung zu einer Mehrzahl Verbinderstifte zur Verbindung mit der Ultraschall-Messausrüstung herzustellen, wobei die Kontaktpunktstege (22a ... 22n) mit einer solchen Form und Größe ausgebildet sind, dass sie die Kontaktstückstege überlappen und an den Kontaktstückstegen angeklemmt sind.

2. Ultraschallsignal-Kabelverbindervorrichtung (10) nach Anspruch 1, bei der die Mehrzahl Signalleitungs-Verbindungsstege (15a ... 15n), Kontaktstückstege (18a ... 18n) und Verdrahtungsmuster (17a ... 17n) sowohl auf der vorderen (11a) als auch auf der hinteren Oberfläche (11b) der rechteckigen Verdrahtungsplatine (11) vorgesehen ist.

3. Ultraschallsignal-Kabelverbindervorrichtung (10) nach Anspruch 1, bei der die Mehrzahl Signalleitungs-Verbindungsstege (15a ... 15n) parallel auf der vorderen (11a) und der hinteren Oberfläche (11b) einer der kurzen Seiten der rechteckigen Verdrahtungsplatine (11) vorgesehen ist, die Mehrzahl Kontaktstückstege (18a ... 18n) in Form von Spalten auf beiden langen Seiten der rechteckigen Verdrahtungsplatine (11) positioniert ist und die auf der hinteren Oberfläche (11b) der rechteckigen Verdrahtungsplatine (11) angeordneten Signalleitungs-Verbindungsstege (15a ... 15n) mit den Kontaktstückstegen (18a ... 18n) über Durchgangsbohrungen (27a, 27b) verbunden sind.

4. Ultraschallsignal-Kabelverbindervorrichtung (10) nach Anspruch 2, bei der der Ultraschallverbinder (68) aufweist:
eine Verbinderbuchse (20) mit
einer Mehrzahl erster Kontaktpunktstege (43a ... 43n), die mit den Kontaktstückstegen (18a ... 18n) auf einer Fläche der rechteckigen Verdrahtungsplatine (11) verbunden sind, und
einer Mehrzahl zweiter Kontaktpunktstege (48a ... 48n), die entlang der Mehrzahl erster Kontaktpunktstege (43a ... 43n) angeordnet sind; und
einer Mehrzahl dritter Kontaktpunktstege (52a ... 52n), die mit den Kontaktstückstegen (18a ... 18n) auf der anderen Fläche der rechteckigen Verdrahtungsplatine (11) verbunden sind,
einer Mehrzahl vierter Kontaktpunktstege (52'a ... 52'n), die mit der Mehrzahl zweiter Kontaktpunktstege (48a ... 48n) der Verbinderbuchse (20) verbunden sind, und
einem Verdrahtungsmuster (54a ... 54n), das die dritten (52a ... 52n) und vierten Kontaktpunktstege (52'a ... 52'n) verbindet.

5. Ultraschall-Endoskop (61), mit:
einem Ultraschallwander (73), der am distalen Ende (69) eines Einführabschnitts vorgesehen ist, der in einen Körperhohlraum einzuführen ist, um eine Ultraschallwelle zum Gewebe des Körperhohlraums zu erzeugen und die vom Gewebe des Körperhohlraums reflektierte Ultraschallwelle zu empfangen, um ein Ultraschallsignal zu erzeugen;
einem Ultraschall-Signalkabel (67) mit einer Mehrzahl Signalleitungen (81), wobei ein Ende mit einer Mehrzahl piezoelektrischer Elemente, die den Ultraschallwandler (73) bilden, verbunden ist und das andere Ende mit einer Ultraschallsignal-Kabelverbindervorrichtung (10) verbunden ist; und
einem Ultraschallverbinder (68), der mit dem Ultraschall-Signalkabel (67) und der Ultraschall-Messausrüstung verbunden ist;
wobei die Ultraschallsignal-Kabelverbindervorrichtung (10) aufweist:
eine rechteckige Verdrahtungsplatine (11), die etwa die gleiche wie oder eine kleinere Breitenabmessung hat als der Außendurchmesser des Ultraschall-Signalkabels (67);
eine Mehrzahl Signalleitungs-Verbindungsstege (15a ... 15n), die parallel auf der Oberfläche einer der kurzen Seiten der rechteckigen Verdrahtungsplatine (11) angeordnet sind und die Mehrzahl Signalleitungen (81) des Ultraschall-Signalkabels (67) elektrisch verbinden;
eine Mehrzahl Kontaktstückstege (18a ... 18n), die in Form einer Spalte in Längsrichtung auf der Oberfläche der rechteckigen Verdrahtungsplatine (11) angeordnet sind, um eine Mehrzahl Anschluss-Verbinderstücke, die für den Ultraschallverbinder (68) vorgesehen sind, elektrisch zu verbinden; und
eine Mehrzahl Verdrahtungsmuster (17a ... 17n) zum Verbinden der Mehrzahl Signalleitungs-Verbindungsstege (15a ... 15n) und Kontaktstückstege (18a ... 18n), die auf der Oberfläche der rechteckigen Verdrahtungsplatine (11) vorgesehen sind;
eine Verbinderbuchse (20), an der die rechteckige Verdrahtungsplatine (11) befestigt ist, wobei die Verbinderbuchse (20) für den Ultraschallverbinder (68) vorgesehen ist; und
eine Mehrzahl Kontaktpunktstege (22a ... 22n), die auf einer Verdrahtungsplatine (21) der Verbinderbuchse (20) vorgesehen sind, eine Mehrzahl Verbindungsmuster (24a ... 24n), die sich von den Kontaktpunktstegen aus erstrecken, um die Verbindung zu einer Mehrzahl Verbinderstifte zur Verbindung mit der Ultraschall-Messausrüstung herzustellen, wobei die Kontaktpunktstege mit einer solchen Form und Größe ausgebildet sind, dass sie die Kontaktstückstege überlappen und an den Kontaktstückstegen (18a ... 18n) angeklemmt sind.

6. Ultraschall-Endoskop (61) nach Anspruch 5, bei dem die Mehrzahl Signalleitungs-Verbindungsstege (15a ... 15n), Kontaktstückstege (18a ... 18n) und Verdrahtungsmuster (17a ... 17n) sowohl auf der vorderen (11a) als auch auf der hinteren Oberfläche (11b) der rechteckigen Verdrahtungsplatine (11) vorgesehen ist.

7. Ultraschall-Endoskop (61) nach Anspruch 5, bei der die Mehrzahl Signalleitungs-Verbindungsstege (15a ... 15n) parallel auf der vorderen (11a) und der hinteren Oberfläche (11b) einer der kurzen Seiten der rechteckigen Verdrahtungsplatine (11) vorgesehen ist, die Mehrzahl Kontaktstückstege (18a ... 18n) in Form von Spalten auf beiden langen Seiten der rechteckigen Verdrahtungsplatine (11) positioniert ist und die auf der hinteren Oberfläche (11b) der rechteckigen Verdrahtungsplatine (11) angeordneten Signalleitungs-Verbindungsstege (15a ... 15n) mit den Kontaktstückstegen (18a ... 18n) über Durchgangsbohrungen (27a, 27b) verbunden sind.

8. Ultraschall-Endoskop (61) nach Anspruch 6, bei dem der Ultraschallverbinder (68) aufweist:
eine Verbinderbuchse (20) mit
einer Mehrzahl erster Kontaktpunktstege (43a ... 43n), die mit den Kontaktstückstegen (18a ... 18n) auf einer Fläche der rechteckigen Verdrahtungsplatine (11) verbunden sind, und
einer Mehrzahl zweiter Kontaktpunktstege (48a ... 48n), die entlang der Mehrzahl erster Kontaktpunktstege (43a ... 43n) angeordnet sind; und
einer Verbindungsplatine (51) mit einer Mehrzahl dritter Kontaktpunktstege (52a ... 52n), die mit den Kontaktstückstegen (18a ... 18n) auf der anderen Fläche der rechteckigen Verdrahtungsplatine (11) verbunden sind,
eine Mehrzahl vierter Kontaktpunktstege (52'a ... 52'n), die mit der Mehrzahl zweiter Kontaktpunktstege (48a ... 48n) der Verbinderbuchse (20) verbunden sind, und
einem Verdrahtungsmuster (54a ... 54n), das die dritten (52a ... 52n) und vierten Kontaktpunktstege (52'a ... 52'n) verbindet.

## Revendications

1. Dispositif de connecteur de câble pour signaux ultrasonores (10) devant être utilisé avec
un dispositif d'endoscope à ultrasons comprenant
un endoscope à ultrasons (61) comportant un transducteur à ultrasons à balayage électronique (73) prévu sur une extrémité distale (69) d'une partie d'insertion devant être insérée dans une cavité de corps, pour générer une onde ultrasonore sur le tissu de la cavité de corps et recevoir l'onde ultrasonore réfléchie depuis le tissu de la cavité de corps de façon à générer des signaux ultrasonores, et
un équipement de mesure à ultrasons destiné à réaliser une commande d'attaque du transducteur à ultrasons (73) de l'endoscope à ultrasons (61) et générer des signaux d'images ultrasonores générés à partir du signal ultrasonore,
le dispositif de connecteur de câble pour signaux ultrasonores (10) comprenant :
un câble pour signaux ultrasonores (67) comportant une pluralité de lignes de signaux (81) adaptées pour être connectées au transducteur à ultrasons à balayage électronique (73) ;
un connecteur à ultrasons (68) destiné à connecter le câble pour signaux ultrasonores (67) à l'équipement de mesure à ultrasons ;
une carte de câblage rectangulaire (11) qui présente approximativement la même dimension de largeur ou moins que le diamètre externe du câble pour signaux ultrasonores (67) ;
une pluralité de plages d'accueil de connexions de lignes de signaux (15a...15n) disposées en parallèle sur la surface de l'un des côtés courts de la carte de câblage rectangulaire (11) connectant électriquement la pluralité de lignes de signaux (81) du câble pour signaux ultrasonores (67) ;
une pluralité de plages d'accueil de pièces de contact (18a...18n) disposées en une colonne longitudinalement sur la surface de la carte de câblage rectangulaire (11) pour connecter électriquement une pluralité de pièces de connecteur de connexion prévues sur le connecteur à ultrasons (68) ;
une pluralité de schémas de câblage (17a...17n) destinés à connecter la pluralité de plages d'accueil de connexions de lignes de signaux (15a...15n) et de plages d'accueil de pièces de contact (18a...18n) qui sont prévues sur la surface de la carte de câblage rectangulaire (11) ;
un socle de connecteur (20) sur lequel la carte de câblage rectangulaire (11) est fixée, le socle de connecteur (20) étant prévu sur le connecteur à ultrasons (68) ; et
une pluralité de plages d'accueil de points de contact (22a...22n) prévues sur une carte de câblage (21) du socle de connecteur (20), une pluralité de schémas de connexion (24a...24n) s'étendant depuis les plages d'accueil de points de contact pour connexion électrique à une pluralité de broches de connecteur pour connexion à l'équipement de mesure à ultrasons, les plages d'accueil de points de contact étant formées selon une forme et une dimension de façon à chevaucher les plages d'accueil de pièces de contact et étant bloquées sur les plages d'accueil de pièces de contact.

2. Dispositif de connecteur de câble pour signaux ultrasonores (10) selon la revendication 1, dans lequel la pluralité de plages d'accueil de connexions de lignes de signaux (15a...15n), de plages d'accueil de pièces de contact (18a...18n), et les motifs de câblage (17a...17n) sont prévus à la fois sur les surfaces avant (11a) et arrière (11b) de la carte de câblage rectangulaire (11).

3. Dispositif de connecteur de câble pour signaux ultrasonores (10) selon la revendication 1, dans lequel la pluralité de plages d'accueil de connexions de lignes de signaux (15a...15n) sont disposées en parallèle sur les surfaces avant (11a) et arrière (11b) de l'un des côtés courts de la carte de câblage rectangulaire (11), la pluralité de plages d'accueil de pièces de contact (18a...18n) sont positionnées en colonnes sur les deux côtés longs sur la surface de la carte de câblage rectangulaire (11), et les plages d'accueil de connexions de lignes de signaux (15a...15n) prévues sur la surface arrière (11b) de la carte de câblage rectangulaire (11) sont connectées aux plages d'accueil de pièces de contact (18a...18n) via des trous traversants (27a, 27b).

4. Dispositif de connecteur de câble pour signaux ultrasonores (10) selon la revendication 2, le connecteur à ultrasons (68) comprenant :
un socle de connecteur (20) comportant
une pluralité de premières plages d'accueil de points de contact (43a...43n) connectées aux plages d'accueil de pièces de contact (18a...18n) prévues sur une face (11a) de la carte de câblage rectangulaire (11), et
une pluralité de deuxièmes plages d'accueil de points de contact (48a...48n) disposées le long de la pluralité de premières plages d'accueil de points de contact (43a...43n) ; et
une carte de connexions (51) comportant
une pluralité de troisièmes plages d'accueil de points de contact (52a...52n) connectées aux plages d'accueil de pièces de contact (18a...18n) prévues sur l'autre face (11b) de la carte de câblage rectangulaire (11),
une pluralité de quatrièmes plages d'accueil de points de contact (52'a...52'n) connectées à la pluralité de deuxièmes plages d'accueil de points de contact (48a...48n) du socle de connecteur (20), et
un schéma de câblage (54a...54n) qui connecte les troisièmes (52a...52n) et quatrièmes (52'a...52'n) plages d'accueil de points de contact.

5. Endoscope à ultrasons (61) comprenant :
un transducteur à ultrasons (73) prévu sur une extrémité distale (69) d'une partie d'insertion devant être insérée dans une cavité de corps, pour générer une onde ultrasonore sur le tissu de la cavité de corps et recevoir l'onde ultrasonore réfléchie depuis le tissu de la cavité de corps de façon à générer un signal ultrasonore ;
un câble pour signaux ultrasonores (67) comportant une pluralité de lignes de signaux (81) dans lequel une extrémité est connectée à une pluralité d'éléments piézo-électriques constituant le transducteur à ultrasons (73), et l'autre extrémité est connectée à un dispositif de connecteur de câble pour signaux ultrasonores (10) ; et
un connecteur à ultrasons (68) connecté au câble pour signaux ultrasonores (67) et connecté à l'équipement de mesure à ultrasons ;
le dispositif de connecteur de câble pour signaux ultrasonores (10) comprenant :
une carte de câblage rectangulaire (11) qui présente approximativement la même dimension de largeur ou moins que le diamètre externe du câble pour signaux ultrasonores (67) ;
une pluralité de plages d'accueil de connexions de lignes de signaux (15a...15n) disposées en parallèle sur la surface de l'un des côtés courts de la carte de câblage rectangulaire (11) connectant électriquement la pluralité de lignes de signaux (81) du câble pour signaux ultrasonores (67) ;
une pluralité de plages d'accueil de pièces de contact (18a...18n) disposées en une colonne longitudinalement sur la surface de la carte de câblage rectangulaire (11) pour connecter électriquement une pluralité de pièces de connecteur de connexion prévues sur le connecteur à ultrasons (68) ; et
une pluralité de schémas de câblage (17a...17n) destinés à connecter la pluralité de plages d'accueil de connexions de lignes de signaux (15a...15n) et de plages d'accueil de pièces de contact (18a...18n) qui sont prévues sur la surface de la carte de câblage rectangulaire (11) ;
un socle de connecteur (20) sur lequel la carte de câblage rectangulaire (11) est fixée, le socle de connecteur (20) étant prévu sur le connecteur à ultrasons (68) ; et
une pluralité de plages d'accueil de points de contact (22a...22n) prévues sur une carte de câblage (21) du socle de connecteur (20), une pluralité de schémas de connexion (24a...24n) s'étendant depuis les plages d'accueil de points de contact (22a...22n) pour connexion électrique à une pluralité de broches de connecteur pour connexion à l'équipement de mesure à ultrasons, les plages d'accueil de points de contact (22a...22n) étant formées selon une forme et une dimension de façon à chevaucher les plages d'accueil de pièces de contact et étant bloquées sur les plages d'accueil de pièces de contact (15a...15n).

6. Endoscope à ultrasons (61) selon la revendication 5, dans lequel la pluralité de plages d'accueil de connexions de lignes de signaux (15a...15n), de plages d'accueil de pièces de contact (18a...18n), et de schémas de câblage (17a...17n) sont prévus à la fois sur les surfaces avant (11a) et arrière (11b) de la carte de câblage rectangulaire (11).

7. Endoscope à ultrasons (61) selon la revendication 5, dans lequel la pluralité de plages d'accueil de connexions de lignes de signaux (15a...15n) sont disposées en parallèle sur les surfaces avant (11a) et arrière (11b) de l'un des côtés courts de la carte de câblage rectangulaire (11), la pluralité de plages d'accueil de pièces de contact (18a...18n) sont positionnées en colonnes sur les deux côtés longs de la carte de câblage rectangulaire (11), et les plages d'accueil de connexions de lignes de signaux (15a...15n) prévues sur la surface arrière (11b) de la carte de câblage rectangulaire (11) sont connectées aux plages d'accueil de pièces de contact (18a...18n) via des trous traversants (27a, 27b).

8. Endoscope à ultrasons (61) selon la revendication 6, le connecteur à ultrasons (68) comprenant :
un socle de connecteur (20) comportant
une pluralité de premières plages d'accueil de points de contact (43a...43n) connectées aux plages d'accueil de pièces de contact (18a...18n) prévues sur une face (11a) de la carte de câblage rectangulaire (11), et
une pluralité de deuxièmes plages d'accueil de points de contact (48a...48n) disposées le long de la pluralité de premières plages d'accueil de points de contact (43a...43n) ; et
une carte de connexions (51) comportant
une pluralité de troisièmes plages d'accueil de points de contact (52a...52n) connectées aux plages d'accueil de pièces de contact (18a...18n) prévues sur l'autre face (11b) de la carte de câblage rectangulaire (11),
une pluralité de quatrièmes plages d'accueil de points de contact (52'a...52'n) connectées à la pluralité de deuxièmes plages d'accueil de points de contact (48a...48n) du socle de connecteur (20), et
un schéma de câblage (54a...54n) qui connecte les troisièmes (52a...52n) et quatrièmes (52'a...52'n) plages d'accueil de points de contact.
